# EUROPEAN PATENT SPECIFICATION

(11) **EP 0 319 815 B1**
(45) Date of publication and mention of the grant of the patent: **31.08.1994**
(21) Application number: 88119774.3
(22) Date of filing: 28.11.1988
(51) Int. Cl.: C12N 5/10, G01N 33/50

(54) **Transfectant cell lines which express the major human rhinovirus receptor**
Transfizierte Zellstämme, die den menschlichen Rhinovirus-Hauptrezeptor exprimieren
Lignées de cellules transfectées exprimant le récepteur principal du rhinovirus humain

(30) Priority: 08.12.1987 US 130378; 25.10.1988 US 262570
(43) Date of publication of application: 14.06.1989
(73) Proprietor: MILES INC., Pittsburgh, PA 15219-2502 (US)
(72) Inventor: McClelland, Alan, Old Saybrook, CT 06475 (US); Meyer, Ann Marie, Branford, CT 06405 (US); Greve, Jeffrey M., Branford, CT 06405 (US); Davis, Gary, Milford, CT 06460 (US)
(74) Representative: Dänner, Klaus, Dr.

(56) References cited:
- EP-A- 0 169 146
- EP-A- 0 169 729
- GB-A- 2 022 826
- CHEMICAL ABSTRACTS, vol. 106, no. 13, 1987, Columbus, Ohio, US; J.E. TOMASSINI:"Isolation, characterization and cloning of the cellular receptor for the majorgroup of human rhinoviruses" & DISS. ABSTR. INT. B 1987, 47(7),2774
- IDEM
- CHEMICAL ABSTRACTS, vol. 96, 1982, page 571, abstract no. 160566z, Columbus,Ohio, US; D. LEBMAN et al.: "A monoclonal antibody that detects expression oftransferrin receptor in human erythroid percursor cells",& BLOOD 1982, 59(3), 671-8
- PROC. NATL. ACAD. SCI., vol. 83, October 1986, pages 7845-7849, US; C.MENDELSOHN et al.: "Transformation of a human poliovirus receptor gene intomouse cells"

## Description

### BACKGROUND OF THE INVENTION

### Field of the Invention

The present invention concerns substantially homogeneous high expression cloned cell lines derived from primary transfectants prepared by gene transfection of human DNA into non-human mammalian cells which express the human rhinovirus receptor gene. The invention also concerns a method for screening compounds for the ability to block binding of a ligand to a human cell surface receptor protein.

### Background Information

The human rhinoviruses are the major causitive agent of upper respiratory tract infections including the "common cold". Approximately 100 immunologically distinct serotypes have been identified and thus a vaccine approach to the prevention of infection is not believed to be feasible. Furthermore, it is difficult to grow rhinoviruses to high titers in culture. In addition, many rhinovirus serotypes are present during single respiratory disease outbreaks and may recur only rarely in the same area. Injection of purified vaccines has shown that the high levels of serum antibody are frequently not associated with similar elevation of local secretory antibody, which may be the most significant factor in disease prevention.

Viral attachment and entry into cells is mediated by specific cell surface receptors. Competition binding studies have shown that 80% or more of rhinovirus serotypes bind to a single receptor which has been termed the "major receptor". Most of the remaining serotypes bind to a second minor receptor.

Rhinoviruses are picornaviruses similar to enteroviruses, but differing from them in having a CsCl buoyant density of 1.40 g/mL and in being acid-labile.

The rhinovirus enters via the upper respiratory tract. High titers of virus in nasal secretions, which can be found as early as 2 to 4 days after exposure, are associated with maximal illness. Thereafter, viral titers fall, although illness persists.

Histopathologic changes are limited to the submucosa and surface epithelium. These include engorgement of blood vessels, edema, mild cellular infiltration, and desquamation of surface epithelium, which is complete by the third day. Nasal secretion increases in quantity and in protein concentration.

The incubation period is brief, from 2 to 4 days and the acute illness usually lasts for 7 days, although a nonproductive cough may persist for 2-3 weeks. The average adult has 1 to 2 attacks each year. Usual symptoms in adults include irritation in the upper respiratory tract, nasal discharge, headache, mild cough, malaise and a chilly sensation. Approximately one-fifth of rhinovirus infections result in fever symptoms. The nasal and nasopharyngeal mucosa become red and swollen and the sense of smell becomes less keen. Mild hoarseness may be present. Prominent cervical adenopathy does not occur. Secondary bacterial infection may produce acute otitis media, sinusitis, bronchitis, or pneumonitis, especially in children. Type-specific antibodies appear or rise with each infection.

Natural immunity may exist, but may be brief. Only 30 to 50% of volunteers can be infected with infectious material; yet the "resistant" volunteers may catch colds of the same serotype at other times. Furthermore, people in isolated areas have more severe colds and a higher incidence of infection when a cold is introduced, than people in areas regularly exposed to the virus. It has also been observed that older adults experience fewer colds than young adults and children. One three year study of acute respiratory tract illness in college and medical students showed that the same serotype was never isolated from separate illnesses in any student who had 2 or more illnesses.

Recent work with human volunteers has shown that resistance to the common cold is independent of measurable serum antibody, but perhaps is related to specific antibody in the nasal secretions. These secretory antibodies are primarily 11S IgA immunoglobulins, produced locally in the mucosa and not a transudate from the serum. These 11S IgA antibodies do not persist as long as those in serum and this could explain the paradox of reinfection in a person with adequate serum antibodies. Results regarding the role of interferon in recovery are inconclusive to date.

Volunteers infected with one rhinovirus serotype resist challenge with both homologous and heterologous virus for 2 to 16 weeks after the initial infection. Resistance to homologous challenge is complete during this period, while resistance to heterologous challenge is incomplete. This nonspecific resistance may be a factor in the control of naturally occurring colds.

The disease occurs throughout the world. In the temperate zones, the attack rates are highest in early fall and winter, declining in the late spring. Members of isolated communities form highly susceptible groups.

Colds in children spread more easily to others than do colds in adults. Adults in households with a child in school have twice as many colds as adults in households without school children.

In a single community, many rhinovirus serotypes cause outbreaks of disease in a single season and different serotypes predominate during different respiratory disease seasons.

The first step in rhinovirus infection of human cells is the attachment of the virus to specific cell surface receptors. The presence of these receptors is an absolute requirement for viral entry and therefore for successful infection of a cell. Rhinovirus receptors have been identified on the surface of human cells in tissue culture using virus binding assays (L. Medrano and H. Green, "Picornavirus Receptors and Picornavirus Multiplication in Human-Mouse Hybrid Cell Lines", Virology, 54, 515-524, (1973); G. Abraham and R. J. Colonno, "Many Rhinovirus Serotypes Share the Same Cellular Receptor", J. Virol., 51, 340-345, (1984)). Rhinovirus infection is restricted to higher primates, particularly humans. This restriction is likely to be due at least in part to the requirement for receptors which are not found on non-human cells with the exception of certain simian species.

Competition binding studies have shown that at least 80% of rhinovirus serotypes bind to a common cellular receptor and that most of the remaining serotypes bind to a second receptor, giving rise to the terms "major" and "minor". receptors. Monoclonal antibodies which recognize the major receptor were isolated by R. J. Colonno, P. L. Callahan and W. J. Long, "Isolation of a Monoclonal Antibody that Blocks Attachment of the Major Group of Human Rhinoviruses", J. Virol., 57, 7-12, (1986) using methods previously described to isolate antibodies to the Coxsackie B virus receptor (Campbell and Cords, J. Virol., 48, 561-564, (1983)) and the poliovirus receptor (Minor et al, Virus Res., 1, 203-232, (1984); P. Nobis, R. Zibirre, G. Meyer, J. Kuhne, G. Warnecke and G. Koch, "Production of a Monoclonal Antibody Against an Epitope of HeLa Cells that is the Functional Poliovirus Binding Site", J. Gen. Virol., 66, 2563-2569, (1985)).

In this procedure, antibodies are raised against cell surface molecules by immunizing mice with cells of another species, in this case human cells which express the receptor of interest. Hybridomas are produced and the supernatants are screened for blocking activity against viral infection of cell monolayers in a standard infectivity assay.

R. J. Colonno, P. L. Callahan and W. J. Long screened approximately 8000 hybridomas and identified a monoclonal antibody which prevented infection of HeLa cells by rhinovirus serotypes which bind to the major receptor. It was found that the antibody showed a similar profile of binding activity to a panel of tissue culture cells as does the virus, i.e., binding to the majority of human cell lines tested as well as to monkey cell lines, but no binding to cell lines from any other species. It was later shown by J. E. Tomassini and R. J. Colonno, "Isolation of a Receptor Protein Involved in the Attachment of Human Rhinoviruses", J. Virology, 58, 290-295, (1986), that this monoclonal antibody immunoprecipitates a 90 kd polypeptide from human cells. The antibody was used for affinity purification of the 90 kd polypeptide and this purified material was used to raise a polyclonal antiserum in rabbits. The rabbit polyclonal antibody was shown to also immunoprecipitate the 90 kd polypeptide and to have blocking activity in the infectivity assay. This correlation between the receptor blocking antibodies and the 90 kd polypeptide led to the conclusion that this was the major human rhinovirus receptor, although not directly proving that this was the case, i.e., it was not shown that the 90 kd polypeptide has rhinovirus receptor activity, but rather that it could be used to elicit an antibody response which had receptor blocking activity. Thus, for example, possibilities such as the association of the 90 kd polypeptide with a second protein which is the true receptor could not be excluded by these data.

A number of attempts have been made to produce monoclonal antibodies that would block attachment of the human rhinovirus to human cells. European Patent Specification No. 169,146 concerns the sequencing of the human rhinovirus serotype HRV 14 and the production of monoclonal antibodies against the viral protein. European Patent Specification 192,175 concerns the sequencing of the HRV2 serotype. McCray and Werner, Nature, 329, 736-738, (1987), prepared monoclonal antibodies against synthetic peptide immunogens comprising peptide sequences postulated from the protein sequence of HRV to form part of the binding site on the virus for the cell surface receptor protein. These studies have failed either by being unable to demonstrate substantial blocking of virus binding to the receptor protein or by the lack of efficacy in human subjects.

Monoclonal antibodies isolated by immunization with HeLa cells and screening for blocking virus attachment were described in C. Mendelsohn, B. Johnson, K. A. Lionetti, P. Nobis, E. Wimmer and V. R. Racaniello, "Transformation of a Human Poliovirus Receptor Gene into Mouse Cells", Proc. Natl. Acad. Sci. U.S.A., 83, 7845-7849, (1986), for the poliovirus receptor.

### SUMMARY OF THE INVENTION

The present invention provides a substantially homogeneous high expression cloned cell line derived from a primary transfectant prepared by gene transfection of human DNA into non-human mammalian cells, which cloned cell line expresses about ten fold or greater more human rhinovirus receptor than HeLa cells.

Accordingly, the invention provides the basis for cloning the human rhinovirus receptor protein gene and for elucidating the sequence and structure of this surface protein. Such information will permit the generation of peptides which can mimic the receptor binding interactions with the virus and thereby serve as effective blocking agents for infection. Moreover, the receptor-expressive non-human cell lines produced by gene transfectant techniques provide improved immunogens for generating antibodies against the receptor protein and a source of expressed receptor protein free of other human proteins.

Primary transfectant cell lines are obtained by gene transfection of human DNA into suitable non-human mammalian cells, e.g., mouse cells. Secondary transfectants, characterized by the virtual absence of any human DNA sequences, other than the gene coding for the receptor protein, can then be obtained by gene transfection of the primary transfectant DNA into suitable non-human mammalian cells. Cloning of secondary tranfectants leads to the isolation of substantially homogeneous high expression cloned cell lines that express as much as ten fold or more human rhinovirus receptor than parent human cells, e.g., HeLa cells.

The rhinovirus receptor gene can be cloned from a secondary transfectant by conventional methods, e.g., the method of Kuehn, McClelland and Ruddle, Cell, 37, 95-103, (1984). Cloned fragments of human genomic DNA which code for the major human rhinovirus receptor can be inserted into appropriate recombinant DNA expression vectors. Cells suitably transformed by such expression vectors will express the major human rhinovirus receptor protein.

The primary and secondary transfectants can be used as immunogens to produce antibodies, particularly monoclonal antibodies, to the receptor. The transfectants provide a more specific immunogen than, for example, HeLa cells because the species of mammalian cells expressing the human surface protein and the species of the immunized animal can be the same, e.g., mouse, yielding an immunogenic response directed specifically only to the foreign protein. Antibodies produced in this manner will be selected for the ability to block viral attachment to cells and will be useful in delineating the viral attachment points on the receptor molecule. This will provide key information leading to identification of candidate peptides for blocking viral infection.

The present invention also provides a general method for screening compounds for the ability to block binding of a ligand, e.g., a virus, to a human cell surface receptor protein. The method involves contacting non-human mammalian cells produced by gene transfection or other suitable means which express the receptor protein with a candidate compound and determining whether blocking occurs. Current methods for screening such drug candidates are generally based on cell culture models. The first step in such prior art methods is to determine non-toxic levels of the candidate compound. Assays are then performed to determine if the normal ligand-receptor interaction has been blocked. In the case of viral infection, viral replication can be followed in a number of ways depending on the particular virus and the assays available. For example, rhinovirus infection can be followed by monitoring the cytopathic effect of the virus. HIV infection can be followed by syncitium formation or reverse transcriptase activity.

The prior art drug screening methods are limited in a number of important aspects. Since compounds are generally tested in cell culture, non-toxic levels of drug candidates must be used. This almost certainly results in negative results from compounds which would have activity at higher doses. If such compounds could be identified, the toxicity problem can be addressed by subsequent chemical modification, or the use of multiple compounds at non-toxic levels which may act synergistically. In the case of viruses, the choice of the cell model is of importance since viral replication may vary between cell lines. Thus, both false positives and false negatives may arise from the use of an inappropriate model. Many of the assays are time consuming and labor intensive, which severely limits the number of compounds which can be tested.

A number of advantages result from the use of non-mammalian cell lines that express the surface receptor of interest. Compounds that are targeted to act outside the cell can be screened. Toxicity problems are eliminated because the assay can be performed on fixed cells. Rapid screening is possible. Using an appropriate format, e.g., an ELISA-type assay on fixed cell monolayers, hundreds of tests can be done in a single day. The use of cloned secondary transfectants exhibiting increased surface receptor expression provides a more sensitive assay with fewer background problems.

### DETAILED DESCRIPTION OF THE INVENTION

The cell lines of the present invention are obtained by gene transfection. Gene transfection is the process by which DNA is introduced into the nucleus of cultured cells and is used with an appropriate selection to obtain cells which retain and express the transfected DNA as part of their genetic material (F. H. Ruddle, M. E. Kamarck, A. McClelland and L. Kuhn, "DNA Mediated Gene Transfer in Mammalian Gene Cloning", Genetic Engineering, Vol. 6, 319-388 (1984)).

In particular, the preparation of cell lines according to the present invention will involve:
(1) Cotransfection of human DNA, e.g., HeLa DNA with a selectable marker, e.g., the HSV tk gene into recipient cells. The transfection procedure and recipient cells should preferably permit the isolation of several thousand independent transfectants, e.g., the calcium phosphate precipitation procedure using Ltk⁻ cells as recipients.
(2) Selection of primary transfectant cells expressing the major rhinovirus receptor on their surface, for example, by fluorescence activated cell sorting. Such cells can be detected by receptor specific reagents, e.g., fluorescence based virus binding assay or anti-receptor monoclonal antibody.
(3) Cotransfection of primary transfectant DNA into recipient cells as in (1) above and selection of expressing cells as in (2) above to obtain secondary transfectants.
(4) Selection of transfectants with a higher level of expression by cell sorting of the highest 1% of cells in the secondary population and subcloning from a substantially homogeneous high expression population.

The human DNA used in the primary transfection can be essentially any human DNA from a source that contains the complete human genome, but preferably will be from a source cell line that is known to express the receptor protein. In the case of HRV, HeLa cells are known ("Assay and Purification of Picornaviruses", Virology - A Practical Approach, B. W. J. Mahy, ed. IRL, Press) which are among the best for growth and propagation of HRV.

The recipient non-human mammalian cells likewise can vary widely, provided that they exhibit some means for selecting transfectants, undergo efficient transfection and do not themselves express the receptor protein of interest. Essentially all tissue culture cell lines have been developed to have a selectable marker which can be used to isolate successful transfectants. For example, the marker neo which confers resistance to G418 can be used in cells sensitive to G418. While some cell types may be inefficient recipients using the conventional calcium phosphate method, alternative methods such as electroporation or protoplast fusion are available. Provided that several thousand independent transfectants can be achieved, any cell type can be used in the role of the recipient. In the case of human receptor proteins, most human cell types and similar cells can not be used because they normally express the desired receptor protein.

Ltk⁻ cells, particularly of the murine variety, are particularly useful as recipient cells. They are deficient in thymidine kinase activity due to a non-reverting mutation in the cellular thymidine kinase gene. HAT media provides a powerful section for tk activity which can readily be restored to Ltk⁻ cells by transfection of the Herpes tk gene. Thus a well characterized, efficient and convenient selection for cells which have become stably transfected is afforded by the use of these cells. Ltk⁻ cells are among the most efficient recipient cells for the calcium phosphate transfection procedure. Frequencies as high as 1 cell in 300 can be obtained. Since at least several thousand independent transfectants are required for successful genomic transfection of a gene, it is preferable to use recipient cells which transfect efficiently. In the case of HRV, Ltk⁻ cells do not express the rhinovirus receptor gene. Since the selection of transfectants uses a virus binding assay, this can be an essential property of the recipient cells. The repetitive DNA sequences in Ltk⁻ cells do not cross hybridize with human repetitive sequences. Human DNA in Ltk⁻ cells can therefore be detected by repetitive sequence probes. Since a main objective of generating the transfectants is the molecular cloning of the gene, it is important that the recipient cells be of a species other than human which meets this criterion.

The present invention also provides a method for producing a monoclonal antibody to the major human rhinovirus receptor by somatic cell hybridization techniques. In general, a monoclonal antibody of the present invention is produced by immunizing a competent host animal with non-human mammalian cells which express the major HRV receptor (prepared by gene transfection as described above), fusing lymphocytes from such host animal which produce antibody to HRV receptor with myeloma cells to form hybridomas, isolating a hybridoma clone which secretes the desired antibody and harvesting the secreted monoclonal antibody. The lymphocytes involved in the hybridization are commonly spleen cells removed from an immunized animal such as a mouse or rat. Preferably, both the lymphocytes and myeloma cells are of murine origin, with murine myeloma cells deficient in the enzyme hypoxanthine guanine phosphoribosyl transferase being particularly useful. For reviews and commentary on the production of monoclonal antibodies by somatic cell hybridization, reference may be made to the following: Lymphocyte Hybridomas, ed. Melchers et al, Springer-Verlag (New York 1978); Nature, 266, 495(1977); Med. Lab. Sci., 36, 329(1979); and Science, 208, 692(1980).

Formed hybridomas which secrete the desired antibody are isolated, usually by cloning on culture media selective for the fused cell hybridomas and determining by appropriate methods an isolated hybridoma which secretes the desired antibody. The homogeneity of the hybridoma cell line is thereafter preferably assured by subcloning the hybridomas selected for their desirable antibody secretion. Harvesting of the secreted monoclonal antibodies is performed by conventional techniques. Proliferation of antibodies can be accomplished by culturing hybridomas in vitro or in vivo, e.g., introducing the hybridoma into an animal such as a mouse and removing antibody-rich ascites fluid.

Lymphocytes producing antibody to HRV receptor can be obtained from various sites, usually the lymph nodes or spleen. Usually, the selected lymphocytes are spleen cells from the immunized animal. Immunization is accomplished by injection of the host animal, e.g., mouse, rat, or goat, at one or more of a variety of sites with the receptor-expressive non-human mammalian cells, preferably primary transfectant cells, normally in admixture with an adjuvant. Further injections are made at the same or different sites at regular or irregular intervals thereafter until it is evident that anti-receptor antibodies are being produced in vivo.

Myeloma cells, that is, malignant cells from primary bone marrow tumors, produce immunoglobulins with unknown specificity and have the ability to propagate in vitro. In contrast, individual lymphocytes characterized by the secretion of immunoglobulins with known specificity, i.e., antibodies capable of binding a known antigen or hapten, are labile under in vitro culturing. A hybridoma, i.e., a hybrid cell formed by fusion of a single lymphocyte with a single myeloma cell, retains the desired immunological specificity of the lymphocyte parent cell and the desired in vitro stability of the myeloma parent cell. Myeloma cells of various animal origin can be used according to the present invention, for example, myeloma cells from mice or rats, however, for reasons of genetic stability it is preferred to fuse lymphocytes and myeloma cells derived from the same animal species and most preferably, from the same strain of such animal species. Murine lymphocytes and myeloma cells are most commonly use, particularly from the BALB/c strain.

Myeloma cells may be of the secreting or nonsecreting type, the former characterized by secretion of immunoglobulin or fragments thereof from the cell into the surrounding medium. Nonsecreting cell lines are preferred since the resulting hybridomas will feature secretion of only the specific immunoglobulin (e.g., IgG, IgE, IgM, IgA or IgD classes) produced by the parent lymphocyte. Moreover, myeloma cells which have been rendered deficient in a particular nucleotide synthetase enzyme are particularly preferred since such a circumstance provides ready means for isolating hybridomas from nonfused lymphocytes and myeloma cells. When grown on a culture medium containing the substrate or substrates for the nucleotide synthetase enzyme in which the myeloma cell is deficient as the only source of nucleotide formation, hybridomas will survive and proliferate since the deficient enzyme will be provided by retained genetic features from the lymphocyte. However, nonfused myeloma cells will die because of enzyme deficiency and nonfused lymphocytes will die due to their inherent in vitro liability. Preferred myeloma cell lines are those deficient in the enzyme hypoxanthine guanine phosphoribosyl transferase (HGPRT) and unable to survive on culture media containing hypoxanthine, aminopterin and thymidine for nucleotide synthesis (such media being conventionally referred to as HAT media - Science, 145, 709(1964). Particular myeloma cell lines which are useful in the present method are conventionally known in the art.

Fusing of the lymphocyte and myeloma cells to form hybridomas is accomplished by conventional means, usually by mixing the cells in the presence of a fusing agent such as sendai virus or polyethylene glycol (PEG). PEG is the preferred fusing agent and is available in commercial lots which vary widely in degree of purity and suitability for in vitro application. Optimal molecular weights for PEG fusing agents have not been established, with molecular weights between about 1000 and 4000 (commercially available as PEG 1000, etc.) being conventionally used.

The mechanism of fusion is generally unknown and yields efficiencies of one hybridoma formed for about every 10⁴ normal cells present. The ratio of the number of lymphocytes to myeloma cells is usually greater than one, more usually 5 or more. Since an unscreened population of lymphocytes is made available to the myeloma cells for fusion, resulting hybridomas will express a distribution of varying antibody secretions. A hybridoma clone, i.e., a population of cells having a common single cell origin, which secretes the desired antibody is isolated by conventional techniques. A common technique involves the division of the fusion mixture into a multiplicity of diluted volumes in the expectation that the majority of resulting volumes which contain hybridomas will contain no more than one. Preferably, the individual volumes will comprise a selective culture medium, e.g., the HAT medium mentioned above, in which only hybridomas will survive. Aliquots of the separate culture fluids are removed after an appropriate incubation period and subjected to an assay for the desired antibodies. Many methods are commonly used in this screening step, including immunoassay procedures such as radioimmunoassay or non-isotopic immunoassays of the homogeneous or heterogeneous type. Culture fluids which are positive for the desired antibody are then usually subcloned, such as by further limiting dilution steps, to assure monoclonal isolation and for stability of the desired hybridoma.

The desired monoclonal antibodies can be harvested by several different methods. In one method, the hybridoma clone is cultured in vitro for an appropriate length of time and aliquots of the culture fluid drawn off to provide monoclonal antibody-rich fractions. Alternatively, the hybridoma clone is injected into or implanted in a host animal, usually a mouse, resulting in in vitro tumor growth and accumulation of large amounts of monoclonal antibody in the ascites fluid which can be appropriately tapped to permit removal of antibody-rich fractions.

The present invention also concerns a general method of screening compounds for the ability to block binding of a ligand, e.g., a virus, such as human rhinovirus, to a human cell surface receptor protein. The process involves contacting a candidate compound, e.g., a peptide or an antibody or fragment thereof, with non-human mammalian cells which express the receptor protein (examples of such cells are (1) cells from a cell line which is a primary transfectant prepared by gene transfection of human DNA into non-human mammalian cells and (2) cells from a cell line which is a secondary transfectant prepared by gene transfection of primary transfectant DNA into non-human malignant cells) and determining whether said candidate compound blocks the binding of the ligand.

The screening method of the present invention can be applied to essentially any ligand/receptor interaction involving a human cell surface receptor protein that can be expressed on transfected non-human mammalian cells. The function of cell surface receptors is to provide a specific interaction between the cell and molecules which exist outside the cell. There are several different roles for receptors. The purpose of the interaction may be delivery of nutrients to the cell, e.g., iron delivery mediated by interaction of the transferrin receptor with transferrin, or cholesterol delivery through LDL and the LDL receptor. The purpose may be clearance of undesirable compounds from the serum, e.g., removal of proteins by the asialoglycoprotein receptor in the liver. The purpose may be delivery of a specific signal to the cell. There are many examples of this including hormones and growth factors such as insulin and EGF, lymphokines such as IL-2, IL-3, B-cell factors and CSF and neurotransmitters such as endorphins and acetylcholine.

In all of these examples it is well established that the ligand and receptor interact in a specific way. For example, binding affinities and saturation binding can be demonstrated. Relatively little is known at present about the precise molecular basis of ligand/receptor interactions, e.g., definition of the binding sites on receptor or ligand is known for only a few examples such as the LDL receptor and its lipoprotein ligand. Virus receptors constitute a special class of receptors, since the normal cellular function of the receptor is distinct from that of the binding virus. Rather, viruses have adapted to utilize or subvert cellular receptors to mediate their entry into cells. In some cases such as the rhinovirus and poliovirus receptors, the normal cellular function of the receptor is unknown. Other virus receptors are already identified molecules such as the CD4 receptor for HIV.

Non-limiting examples of ligand/receptor situations where a therapeutic blocking agent would be useful include the following:
(1) CD4/HIV receptor
(2) C3d receptor for EBV
(3) Nicotinic acetylcholine receptor
(4) IL-2 receptor
(5) Interferon receptors, alpha, beta, gamma
(6) NGF receptor
(7) EGF receptor
(8) PDGF receptor
(9) Muscarinic acetylcholine receptor
(10) alpha-adrenergic receptor
(11) beta-adrenergic receptor
(12) Substance K receptor
(13) TGF alpha and beta receptors

Candidate compounds that can be screened using the present method can be virtually any compound of potential therapeutic interest. The ability of the present method to accommodate a high testing volume allows more flexibility in selecting candidate compounds. The candidate compounds can be selected totally at random from available compounds or can be targeted by virtue of some structural characterisitic of known or suspected significance.

The blocking of the binding of the ligand to the receptor protein expressed on the surface of the non-human mammalian cells can be determined by adding a labeled form of the ligand or an analog thereof and detecting the extent to which the labeled ligand binds to the mammalian cells. Such labelling can be accomplished by conventional means, such as by using radiolabels, enzymes, dyes, fluorescent compounds, biotin-avidin complexes, etc. Such blocking of the binding of the ligand to the receptor protein expressed on the surface of the non-human mammalian cells can also be determined by adding the ligand or an analog thereof and detecting the binding of ligand or analog to the mammalian cells by adding an antibody to the ligand or analog and detecting the extent to which the antibody binds to the ligand or analog on the mammalian cells.

A particularly rapid and efficient method uses formaldehyde fixed monolayers of the receptor-expressive transfectant cells in microtiter plates. Unlabelled ligand is bound to the cells at 37°C in the presence of a test compound for one hour. Unbound ligand is removed by washing and the cells are incubated for 30 minutes at 4°C with a mouse monoclonal antibody against the ligand. Unbound antibody is removed by washing and the mouse monoclonal antibody binding is quantitated using an anti-mouse monoclonal antibody coupled to a conventional detection system, e.g., a radioactive label, biotin-avidin, a fluorescent label, or an enzyme, such as alkaline phosphatase or horse radish peroxidase (i.e., standard ELISA methods).

The present invention will now be illustrated, but is not intended to be limited, by the following examples.

### EXAMPLES

### Example 1: Gene Transfection

The gene transfection method involved the introduction and stable integration of human genomic DNA into mouse Ltk⁻ cells (thymidine kinase deficient mouse L cells) by calcium phosphate co-precipitation with the HSV (herpes simplex virus) TK gene. Transfected cells were stained with a fluorescent probe specific for the surface molecule of interest and positive cells were enriched from the total population using a Fluorescence Activated Cell Sorter (FACS). This procedure was used to isolate Ltk⁻ cells which express the major rhinovirus receptor gene derived by transfection with HeLa cell genomic DNA.

Specifically, mouse Ltk⁻ cells were co-transfected with HeLa genomic DNA and the HSV TK gene using the standard calcium phosphate procedure and HAT selection (Graham and Van der Eb, Virology, 52, 456-467, (1973)).

### Example 2: Detection of Cells Expressing the Rhinovirus Receptor

In order to detect cells expressing the rhinovirus receptor, two assay systems for fluorescent labeling of the receptor on the surface of live cells were employed. Both systems were optimized using human HeLa cells which express the receptor, then applied to the analysis and preparative cell sorting of transfected mouse cells.

The first method employed ultraviolet (uv) inactivated purified HRV 14 which was incubated with cells in suspension. After removing unbound virus, the cells were incubated first with a monoclonal antibody specific for HRV 14 and then with a commercially available fluorescein conjugated goat anti-mouse IgG. Specific labeling of HeLa cells in the presence of virus, but not in its absence, was obtained indicating that the assay detects the virus receptor. Furthermore, mouse Ltk⁻ cells which are known not to express the receptor give background levels of fluorescence, confirming the specificity of the assay.

The second method was to label the receptor directly using a mouse monoclonal antibody directed against the major receptor. This monoclonal antibody was obtained by immunizing mice with HeLa cells and producing hybridomas from the spleens of those mice. Culture supernatants from the hybridomas were then tested for a protective effect against HRV 14 infection of HeLa cells. Four cloned hybridoma cell lines which could completely protect HeLa cells from infection with a high input of HRV 14 for 3 to 5 days were obtained. The cell lines and corresponding antibodies were designated "c78.1", "c78.2", "c78.4" and "c78.5". The specificity of the antibodies for the major receptor was confirmed by the finding that they had a comparable protective effect against HRV 3 infection (major receptor serotype), while no protection was seen in the case of HRV 2 infection (minor receptor serotype).

HRV 14 was grown on confluent monolayers of HeLa cells and virus was released from the cells by three freeze-thaw cycles. Cellular debris was removed by centrifugation and the virus was concentrated by polyethyleneglycol(PEG) precipitation.

Specifically, the virus from Example 1 was partially purified by sucrose gradient centrifugation and was uv irradiated to reduce infectivity.

5 x 10⁻⁶ transfected cells were stained using the virus binding assay as follows: saturating amounts of HRV 14 were incubated with the cells at 37° C for 30 minutes and then unbound virus was removed and the cells were chilled to 4°C. The cells were sequentially incubated with an anti-HRV 14 mouse monoclonal antibody and with fluoresceinated goat anti-mouse IgG. Sterile cell sorting was performed using a Becton Dickinson FACS IV, with sorting parameters which selected the brightest 1.5% of the cells. Sorted cells were expanded and re-sorted until analysis showed at least 95% positive cells. Single cell cloning was used to establish clonal cell lines which were shown to be 100% positive for expression of the receptor.

### Example 3: Isolation of Mouse Ltk⁻ Cell Transfectants

The isolation of mouse Ltk⁻ cell transfectants was initiated using the indirect virus binding assay described above. A pool of Ltk⁻ cells transfected with HeLa DNA and representing 130,000 independent events was subjected to preparative cell sorting. After 4 rounds of cell sorting, 95% of the cells were positive, showing specific fluorescence in the virus binding assay. Single cell cloning was used to isolate 11 cloned cell lines which were 100% positive for virus specific surface fluorescence. These cell lines were designated HRR 1 through HRR 11. HRR 1 has been deposited with the American Type Culture Collection (ATCC) and has received the designation ATCC CRL 9593, deposited on November 19, 1987. DNA was extracted from each of the lines and analysed by Southern blotting and hybridization to a HeLa DNA probe. This experiment confirmed that each of the cell lines contained human DNA. It was also concluded that several independent transfectants had been obtained, since a number of different hybridization patterns were observed. As expected for primary transfectants, each cell line contained a large amount of human DNA, estimated to be several thousands of kilobases, based on hybridization intensities.

Specifically, to obtain secondary transfectants, DNA from primary transfectant cell line HRR 1 was co-transfected into Ltk⁻ cells and cell sorting was performed as described above, with the exception that monoclonal antibody c78.4 was used for receptor staining. A total of 13 clonal secondary cell lines designated HRR 1.1 to HRR 1.13 were obtained. High expressing cell lines HE 1 to HE 4 were isolated by repeated rounds of sorting of the secondary pool until a homogenous level of expression was obtained, followed by subcloning. HE 1 has been deposited with the ATCC and has received the designation ATCC CRL 9592, deposited on November 19, 1987.

### Example 4: Preparation of Monoclonal Antibodies to the Major HRV Receptor

The Ltk⁻ cells which were transfected with HeLa DNA to isolate rhinovirus receptor transfectants are derived from. a hybrid mouse strain designated C3H/B16. Immunization of C3H/B16 mice with transfectants thus provides a high degree of specificity of the immune response for the human receptor. This is because the majority of antigens, both surface and internal, which are presented by the immunizing cells are "self" and will not elicit an immune response. For this reason the use of transfected cells as immunogens is a better method than the use of cells of another species, such as HeLa cells which will present a wide range of foreign antigens to the mouse.

C3H/B16 mice were immunized by intraperitonal injection of 10-7 primary transfectant HRR7 cells in PBS at three week intervals. Between one and two months after the third immunization, the mice were boosted with a final dose of 10-7 HRR7 cells. Three days later spleen cells were removed and fused with Ag 8 myeloma cells as described. Supernatants from hybridoma cultures were tested for protective effects against infection of HeLa cell monolayers by HRV 3. This was done by adding 100 ul of hybridoma culture supernatant to semi-confluent monolayers of 20,000 HeLa cells in 96 well microtiter plates. The antibody was incubated with the cells for one hour at 37°C and then unbound antibody was removed by washing. 20,000 PFU of virus was added to each well and the plates were incubated at 34°C for 48 hours. Wells were scored using a light microscope for the presence or absence of viral cytopathic effect. Three hybridomas were identified which protect HeLa cells from HRV 3 infection. Each antibody immunoprecipitates the 95 kd rhinovirus receptor protein. A representative hybridoma has been deposited with the American Type Culture Collection and designated ATCC HB 9594, deposited on November 19, 1987.

### Results

The following factors indicate that the human rhinovirus receptor ("HRR") cell lines of the present invention contain and express the major human rhinovirus receptor gene:
(1) The cell lines were isolated using an assay for the specific binding of HRV 14 (human rhinovirus) to the cell surface. This binding requires expression of the major human receptor.
(2) The monoclonal antibody c78.4 binds specifically to the surface of the HRR cell lines, while showing no specific reactivity with the parental L-cells.
(3) Purified ³⁵S labeled HRV 14 binds directly to HRR cells. Levels of binding are similar to HeLa cells and approximately 100 fold higher than the non-specific background binding seen on L-cells.
(4) Binding of HRV 14 to HRR cells can be competed by pre-incubation with monoclonal antibody c78.4. An unrelated monoclonal antibody (anti-transferrin receptor) has no effect.
(5) Monoclonal antibody c78.4 immunoprecipitated a 95 kd polypeptide from HeLa cells. This agrees exactly with the published molecular weight of the major rhinovirus receptor. A 95 kd polypeptide can also be immunoprecipitated from HRR cells, but not from Ltk⁻ cells.
(6) Immunization of mice with HRR cells has been used to produce monoclonal antibodies which protect HeLa cells from HRV 14 infection. These monoclonals immunoprecipitate a 95 kd polypeptide from HeLa cells, which is the major rhinovirus receptor.

Primary transfectant HRR 1 was used to obtain secondary transfectants which express the major receptor gene. The main purpose of this is to eliminate most of the human DNA sequences present in the primary transfectants in order to facilitate molecular cloning of the gene. HRR 1 DNA was co-transfected into L-cells to produce approximately 250,000 tranfectants which were sorted using monoclonal antibody c78.4. After two rounds of cell sorting 95% of the cells were positive for expression and single cell cloning was performed to produce cell lines HRR 1.1 through HRR 1.13 (13 cell lines). All of these lines express the receptor at levels comparable to HeLa cells and contain a limited amount of human DNA as determined by Southern hybridization to a HeLa probe. The secondary transfectants are therefore suitable material for the isolation of the receptor gene.

During the course of secondary transfectant isolation it was observed that the level of expression in the uncloned positive population was heterogeneous, with some cells showing a higher level of fluorescence than HeLa cells. Several cell sorts were performed to isolate the highest expressors from this population. Four cloned cell lines were obtained, designated HE-1 to HE-4, which show a level of surface fluorescence consistent with 5 to 10 fold higher levels of the receptor than is found on HeLa cells. Quantitation of receptor numbers on HE-1 cells by ¹²⁵I labeled antibody binding and immunoprecipitation confirms that they contain 10 times as much receptor as HeLa cells, despite having a smaller surface area. The molecular basis for the high receptor expression is unknown.

Purified protein was then sub]ected to limited or complete proteolytic degradation, peptides were purified by either reverse-phase chromatography, gel filtration, or SDS-PAGE, and then subjected to automated protein sequencing. These sequences were used to search protein sequence (NRFB and MIPSX) and DNA sequence (Genbank) databases. A match of all known peptide sequences determined from HRR protein was made. (Intercellular Adhesion Molecule-1 Simmons et al, "ICAM, An Adhesion Ligand of LFA-1, Is Homologous To The Neural Cell Adhesion Molecule of NCAM", Nature, 331, 624-627 (1988)). ICAM was under investigation by other researchers because of its role in the adhesion of T lymphocytes to a variety of different cell types. It is hypothesized that ICAM (present on fibroblasts, epithelial cells, leukocytes, and endothelial cells) interacts with a structure called LFA-1 (lymphocyte-function associated antigen-1) present on the surface of T lymphocytes, and is thereby responsible for the adhesion to these cell types.

Applicants had determined the sequence of 106 amino acids of the rhinovirus receptor, and all 106 matched exactly the sequence of ICAM (out of a total of 507 amino acids predicted for the ICAM sequence). Other biochemical information supports the identity of HRR with ICAM. First, the primary mRNA translation product synthesized in an in vitro translation system has an apparent molecular weight of 55,000 daltons which is the same as ICAM. Secondly, the HRR protein species found in cells poisoned with tunicamycin, a specific inhibitor of asparagine-linked glycosylation, has an apparent molecular weight of 54,000 daltons, consistent with the removal of a signal sequence from the N-terminus of the protein. Third, partial digestion of core-glycosylated HRR protein indicates the presence of seven asparagine-liked carbohydrate groups, consistent with the presence of eight potential carbohydrate acceptor sequences (N-S/T) in the amino acid sequence of ICAM. Finally, the chromosome map position of HRR was determined to be human chromosome 19, identical to that determined for ICAM.

Since the complete nucleotide and amino acid sequence of ICAM has been detemined, and there is substantial, if not overwhelming evidence that ICAM and the HRR are the same or very similar molecules, the complete amino acid sequence of the rhinovirus receptor is now known. The determination of this amino acid sequence, which is a partial chemical structure of this molecule, provides the ability to design and produce large amount of receptor protein, fragments, functional domains, and truncated versions, and analogs of receptor protein, and peptides that have inhibitory activity towards rhinovirus and coxsackie A virus infection. The complete amino acid sequence also provides information needed for biophysical and biochemical studies of rhinovirus-receptor interaction which will lead to the identification of crucial molecular contacts, which can be used for design of novel inhibitory molecules.

Since the ICAM molecule is a member of the immunoglobulin supergene family that maps to chromosome 19, (Eur. J. Immunol., 15, 103-106 (1984)) and since other picornaviruses, such as poliovirus and coxsackie virus, bind to receptors whose genes are located on chromosome 19, it is posible that ICAM can be used as a basis for the development of therapeutics to counter infections by those other picornaviruses as well. It is possible that ICAM or fragments thereof would be useful directly as therapeutics for other viruses and inflammatory diseases. Alternatively, knowledge of ICAM structure will be useful in the identification of the receptors of those viruses. Further, ICAM-1 is closely related to two adhesion proteins of the adult nervous system, neural cell adhesion molecule (NCAM) and myelin-associated glycoprotein (MAG) and a family of epithelial cell molecules including CEA, NCA, TM-CEA, and the pregnancy-specific B1-glycoproteins. NCAM, MAG and ICAM-1 each have five immunoglobulin-like domins, see Dustin et al "Supergene Families Meet In The Immune System", Commentary, Elsevier Publications, Cambridge, 1988. The relationship of the picornaviruses and the supergene family of ICAM, NCAM and MAG provide the basis of developing proteins, protein fragments, functional domains, analogs and mixtures thereof for inhibiting infectivity of this class of viruses.

Knowledge of the amino acid sequence, and information about the ICAM protein coupled with the knowlege of HHR and rhinovirus provide the basis for the following approaches to design protein fragments and analogs for treatment of rhinovirus infection and for treatment of inflammation.

Soluble forms of biologically active host cell protein could be used to inhibit virus infection, in contrast to the cell membrane bound receptor protein that normally facilitates the infection. Soluble forms of biologically active receptor protein, protein fragments, functional domains or analogs could include use of detergents as described supra. Alternatively, elimination of the C-terminus could render the protein(s) soluble. A biologically active tryptic fragment is a mixture of two species, one with an apparent molecular weight of 83,000 daltons and one of 90,000 daltons (relative to HRR of 95kd). The N-terminus of both species is blocked, indicating that they start from residue 1 of the intact HRR molecule, and peptides are removed from C-termius: the largest possible fragment would be from residue 1 to residue 488. The downward shift in apparent molecular weight relative to intact HRR indicates a loss of > 5,000 daltons, or 45 amino acid residues, which would place the new C-termini of fragments at positions proximal (N-terminal) to the transmembrane segment.

Examples of soluble fragments could include the entire extracellular domain (up to a.a. 480) or could include either/or both distinct parts of the extracellular domain (a.a 1-200; 200-460) of the amino acid sequenc of the receptor protein. It is further anticipated that smaller peptide fragments may provide biologically active analogs for inhibiting virus infection.

A full length cDNA clone of the HRR will be isolated from a cDNA library of He1 or other cells expressing the receptor by screening with oligonucleotides made from the published sequence of ICAM-1. Construction and expression of domain fragments of the HRR will be achieved using established recombinant DNA methodologies (Fisher et al, Nature, 331, 76-78 (1988); Hussey et al, Nature, 331, 78-81 (1988); Deen et al, Nature, 331, 82-86 1988)). A soluble extracellular domain will be made by cleaving a cDNA clone of the HRR coding sequence with ThaI which cuts at position 37 in the signal peptide region and at position 1415, 12 amino acids before the start of the transmembrane domain. Synthetic oligonucleotide linkers will be added in a stepwise fashion to the 5′ and 3′ ends of the molecule to restore the signal peptide and initiator ATG at the N termnus and to introduce an in frame translational stop codon at the C-terminus. The position of the stop condon may be varied to produce alternative truncated forms of the molecule. Similarly, different infrequently cutting restriction enzymes will be used to insert stop condons in other regions of the molecule. Restriction enzyme sites will be included at the ends of the linkers to allow directional cloning into a variety of expression vectors. Oligonucleotide site directed mutagenesis, using conventional methods, will be used to introduce restriction enzyme sites where no convenient naturally occurring sites exist. Additionally, the polymerase chain reaction (PCA) technique will be used to produce specific DNA fragments encoding domains and other subregions of the molecule.

The approach described above will also be used to produce additional subfragments of the receptor such as the five immunoglobulin-like domains (residues 1-88, 89-185, 186-284, 285-385, 386-453, Staunton et al, Cell, 52, 925-933 (1988)). In this case appropriate signal sequences to direct protein secretion for the expression system being used will be included. Various expression systems will be used including viral promoters in mammalina cells (Cate et al, Cell, 45, 685-698 (1986)), insect cells (Smith et al, Pros. Acad. Sci. U.S.A., 82, 8404-8408 (1985)); and E. coli (Skerra and Pluckthun, Science, 240, 1038-1041 (1988)). Subfragments of the receptor produced in the above mannor will be tested for the ability to bind major rhinovirus serotypes and to reduce virus infectivity. Expression of the extra-celluar domain as described above will also be used to derive sufficient quantities of the soluble receptor for structural studies such as X-ray crystallography.

Additional biologically active fragments will be evalutated utilizing overlapping sets of synthetic peptides of 10-20 residues corresponding to part or all of the HRR protein. The peptides will be made and individually tested for the ability to inhibit virus binding to receptor.

These peptide fragments could be direct copies of a portion of the rhinovirus receptor, or could contain sequences from non-contiguous regions of the receptor.

ICAM has been predicated, based on homology to NCAM, to be a member of the immunoglobulin gene superfamily. One would expect that the immunoglobulin-like domains in ICAM would have the basic "immunoglobulin fold", as has been shown for two other members of this family, beta-2-microglobulin and the HLA-A2 alpha-3 domain. This fold consists of a "beta-barrel" conformation consisting of two antiparallel beta-pleated sheets, one composed of three and one composed of four beta strands; a disulfide bond between two cysteine residues (separated by approximately 60 amino acids along the chain) connects the two sheets (Williams, A.F., Immun. Today 8, 298-303 (1987)). Two of the disulfide bonds, those corresponding to domains 2 (C110-C161) and 3 (C212-C265), have been experimentally determined by applicants, providing support for the model. This model for the structure provides a basis for designing unique analogs that could mimic the virus binding site and be useful as receptor blockers. Each pair of antiparallel beta strands in the beta-barrel is linked by a hairpin turn of variable size; such turns or loops that protrude from secondary structures are often found to play roles in recognition of ligands (Lezczynski and Rose, Science, 224, 849-855 (1986)). Such protruding structures may be of particular interest in the rhinovirus receptor, since the receptor binding site on the virus capsid is prosposed to be in a recessed cavity. Using the sequence of the HRR, such turns and loops could be predicited based on a beta-barrel structure and produced as synthetic peptides with addition of novel cysteine residues at the N- and C-terminus of the peptides; a disulfide bond would then be formed between such residues on the same peptide to close the loop covalently (in contrast to the native protein, wherein the loop would be closed by noncovalent interactions between the adjacent beta-strands). Such peptides would have a conformation more analogous to the conformation in the native protein than a simple linear peptide, and would be tested for virus-binding activity.

Method of localizing the region or domain of the molecule responsible for virus-binding activity: Site-directed antibodies directed against specific portion of the HRR (predicted from a working model based on an immunoglobulin fold) could be produced by making synthetic peptides corresponding to selected regions of the protein, coupling such peptides to larger carrier proteins, and immunizing rabbits or other animals with such conjugates by standard methodology. Such antibodies could be tested for the ability to inhibit virus binding; inhibition with a subset of such antibodies would direct attention to specific domains or parts of domains.

Specific reactive groups on some amino acid residues on the receptor protein can be chemically modified under non-denaturing conditions. As a consequence of the modification of some residues virus-binding ability may be lost. By the use of radioactive tracers in the modifying reagent, the modification of some amino acid residues may be correlated with loss of binding activity, implicating those groups in recognition. This would direct attention towards a specific part of the molecule or a specific amino acid residue as playing a specific role in virus binding. Such residues could then be experimentally modified in in vitro mutagenesis experiments. As an example, it has been found that labelling HRR with radioactive Bolton/Hunter reagent (an N-hydroxysuccinimide ester, which specifically modifies N-termini and lysine residues) substantially reduces its ability to bind to rhinovirus.

Determination of the three dimensional structure of the virus-binding domain of the HRR by X-ray crystallography and/or Nuclear Magnetic Resonance: Using the three-dimensional coordinates of HRV14 (from the Brookhaven Data Bank), find the optimal "docking" of the two molecules by computer graphics methodology. The structure of the "docked" complex could then be used to refine and improve the properties of the protein or peptide fragment of the receptor. Examples of such improvements would be: (1) increasing the affinity of virus-binding reaction; (2) producing a smaller molecule; and (3) deleting or damaging other regions of the molecule, such as that needed for binding to LFA-1. If the binding site for LFA-1 is on a different domain, the domain could be deleted. Alternatively, if the binding site for LFA-1 is on the viurs-binding domain, site directed mutagenesis of specific amino acids could be used to inhibit the ability to binding.

Key residues of the receptor involved in virus binding will be determined by oligonucelotide site directed mutagenesis. For example, pools of mutants produced by saturation mutagenesis will be screened by the method of Peterson and Seed (Cell, 54, 65-72 (1988)), using either HRV14 or monoclonal antibody/complement killing as the negative selection, and a rabbit polyclonal antibody as the positive selection. Synthetic peptides corresponding to regions of the molecule identified in this way will be made and tested for virus binding and the ability to reduce infectivity.

Pharmaceutical preparations of proteins, protein fragments, functional domain and analogs have an application in a plurality of diseases. With the knowledge that HRV and LFA-1 both bind to ICAM it is anticipated that analogs of ICAM could be designed that bind to rhinovirus and thereby inhibit rhinovirus infection, but which do not disrupt the interaction of ICAM and LFA-1. Alternatively, mitogenesis of selected residues (amino acids) will be made based on structural predictions and biochemical structure.

Again with the knowlege that ICAM and HRR are the same molecule, it is anticipated that it may have application in fragment, functional domains or analogs of LFA-1 could be utilized to disrupt interactions between HRR and rhinovirus and thereby treat rhinovirus infections.

HRR or fragment of it may have application in the disruption of interactions between ICAM and LFA-1, which could be useful for the treatment of inflammation.

Peptides derived from the known capsid proteins of rhinovirus could be useful for the disruption of interactions between ICAM and LFA-1, which could be useful for the treatment of inflammation. Carbohydrate groups that are not necessary for biological activity will be removed to enhance production of peptides in bacteria.

Site-directed mutagenesis of cystines may be useful to limit refolding to biologically active conformations.

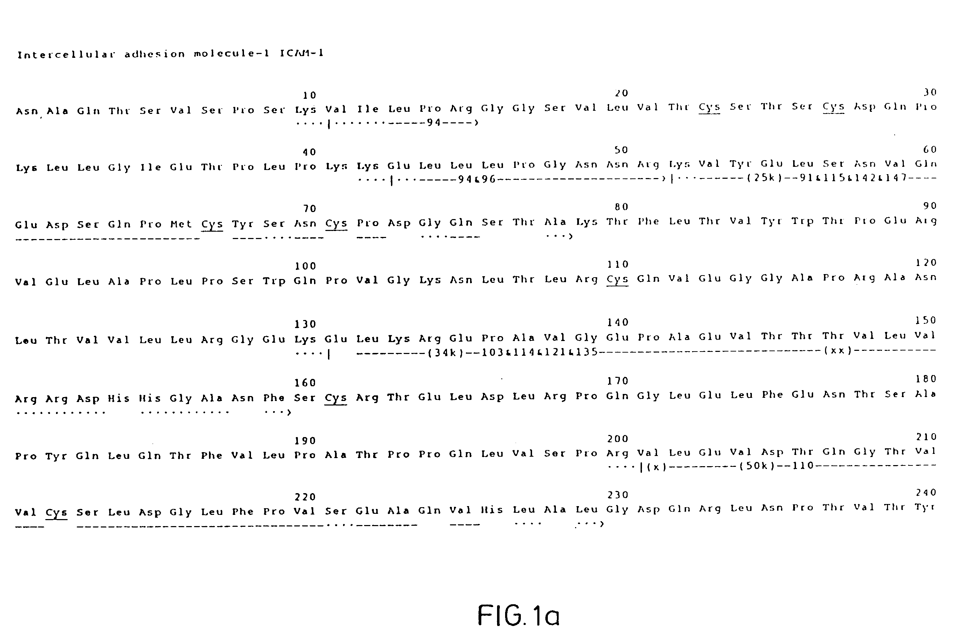

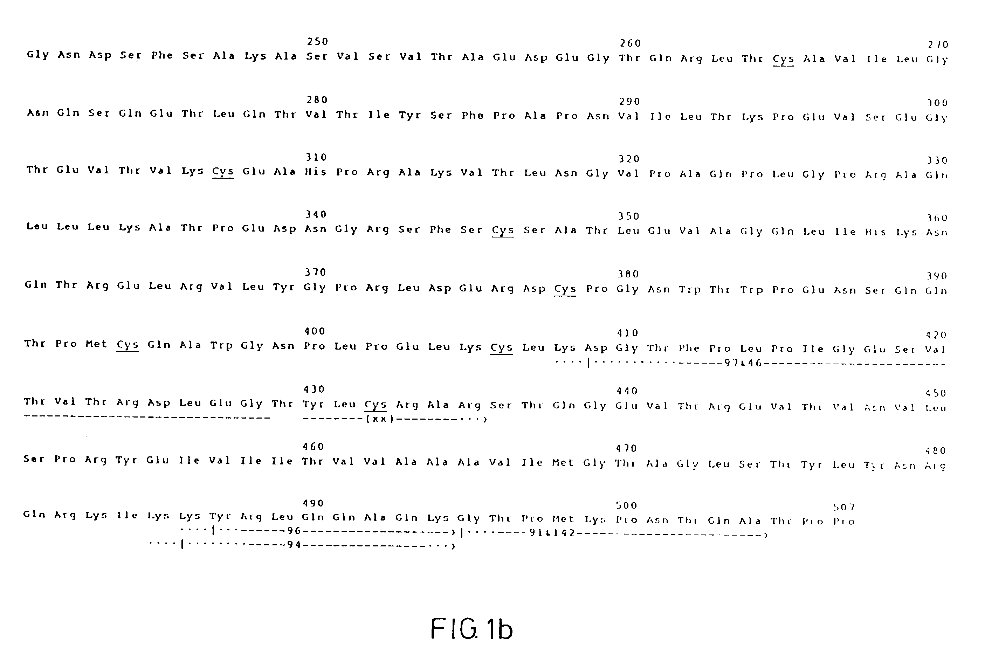

Figures 1a + 1b: Amino acid sequence of ICAM (minus) signal sequence). Sequences obtained from peptide fragments of HRR are indicated as dotted or dashed lines under corresponding sequence of ICAM; dashed means confidently assigned peptide sequences, dotted means ambiguous assignments, and xx means incorrect determinations of ambiguous assignments. The numbers under peptide sequences indicate code name of protein sequencing experiment.

It will be appreciated that the instant specification and claims are set forth by way of illustration and not limitation and that various modifications and changes may be made without departing from the scope of the present invention.

## Claims (Claims for the following Contracting State(s): AT, BE, CH, LI, DE, FR, GB, IT, NL, SE)

1. A substantially homogeneous high expression cloned cell line derived from a primary transfectant prepared by gene transfection of human DNA into non-human mammalian cells, which cloned cell line expresses about ten fold or greater more human rhinovirus receptor than HeLa cells.

2. A method of screening compounds for the ability to block binding of a human rhinovirus of the major receptor group to a cell surface comprising the major rhinovirus receptor, said method comprising contacting cells of claim 1 with a candidate compound and determining whether said candidate compound blocks the binding of said rhinovirus to said cells of claim 1.

3. A method according to claim 2 wherein the candidate compound is selected from the group comprising a peptide, an antibody or a fragment thereof.

## Claims (Claims for the following Contracting State(s): ES)

1. A method of screening compounds for the ability to block binding of a human rhinovirus of the major receptor group to a cell surface comprising the major rhinovirus receptor, said method comprising contacting substantially homogeneous high expression cloned cell lines derived from a primary transfectant prepared by gene transfection of human DNA into non-human mammalian cells, which cloned cell lines express about ten fold or greater more human rhinovirus receptor than HeLa cells with a candidate compound and determining whether said candidate compound blocks the binding of said rhinovirus to said cells.

2. A method according to claim 1 wherein the candidate compound is selected from the group comprising a peptide, an antibody or a fragment thereof.

## Patentansprüche (Patentansprüche für folgende(n) Vertragsstaat(en): AT, BE, CH, LI, DE, FR, GB, IT, NL, SE)

1. Eine klonierte, im wesentliche homogene Hochexpressionszellinie, abgeleitet von einer primären Transfektante, die durch Gentransfizierung menschlicher DNA in nicht-menschliche Säugerzellen hergestellt wurde, wobei die klonierte Zellinie ca. zehnfach oder mehr menschlichen Rhinovirus-Rezeptor als HeLa-Zellen exprimiert.

2. Verfahren zum Screenen von Verbindungen auf die Fähigkeit zur Bindungsblockierung eines humanen Rhinovirus der Hauptrezeptorgruppe an eine Zelloberfläche, die den Rhinovirus-Hauptrezeptor enthält, wobei das Verfahren das Inkontaktbringen der Zellen aus Anspruch 1 mit einer Testverbindung und die Bestimmung, ob die Testverbindung die Bindung des Rhinovirus an die Zellen nach Anspruch 1 blockiert, umfaßt.

3. Verfahren nach Anspruch 2, worin die Testverbindung ausgewählt ist aus einem Peptid, einem Antikörper oder einem Fragment davon.

## Patentansprüche (Patentansprüche für folgende(n) Vertragsstaat(en): ES)

1. Verfahren zum Screenen von Verbindungen auf die Fähigkeit zur Bindungsblockierung eines menschlichen Rhinovirus der Hauptrezeptorgruppe an eine Zelloberfläche, die den Rhinovirus-Hauptrezeptor enthält, wobei das Verfahren das Inkontaktbringen von klonierten, im wesentlichen homogenen Hochexpressionszellinien, abgeleitet aus einer primären Transfektante, hergestellt durch Gentransfektion menschlicher DNA in nicht-menschliche Säugerzellen, wobei die klonierte Zellinien ca. zehnfach oder mehr menschlichen Rhinovirus-Rezeptor als HeLa-Zellen exprimieren, mit einer Testverbindung, und Bestimmen, ob die Testverbindung die Bindung des Rhinovirus an die Zellen blockiert, umfaßt.

2. Verfahren nach Anspruch 1, worin die Testverbindung ausgewählt ist aus einem Peptid, einem Antikörper oder einem Fragment davon.

## Revendications (Revendications pour l'(les) Etat(s) contractant(s) suivant(s): AT, BE, CH, LI, DE, FR, GB, IT, NL, SE)

1. Lignée de cellules clonées a haut degré d'expression, pratiquement homogène, dérivée d'un transfectant primaire préparé par transfection génique d'ADN humain dans des cellules d'un mammifère autre que l'homme, ladite lignée de cellules clonées exprimant le récepteur du rhinovirus humain en une quantité égale ou supérieure à approximativement 10 fois la quantité exprimée par les cellules HeLa.

2. Procédé de sélection de composés pour l'aptitude à bloquer la liaison d'un rhinovirus humain, du groupe correspondant au récepteur principal, à une surface cellulaire comprenant le récepteur principal de rhinovirus, ledit procédé comprenant la mise en contact de cellules suivant la revendication 1 avec un composé à l'essai et l'étape consistant à déterminer si ledit composé à l'essai bloque la liaison dudit rhinovirus auxdites cellules suivant la revendication 1.

3. Procédé suivant la revendication 2, dans lequel le composé à l'essai est choisi dans le groupe comprenant un peptide, un anticorps ou un de ses fragments.

## Revendications (Revendications pour l'(les) Etat(s) contractant(s) suivant(s): ES)

1. Procédé de sélection de composés pour l'aptitude à bloquer la liaison d'un rhinovirus humain, du groupe correspondant au récepteur principal, à une surface cellulaire comprenant le récepteur principal de rhinovirus, ledit procédé comprenant la mise en contact de lignées de cellules clonées à haut degré d'expression, pratiquement homogènes, dérivées d'un transfectant primaire préparé par transfection génique d'ADN humain dans des cellules d'un mammifère autre que l'homme, lignées de cellules clonées qui expriment une quantité de récepteur du rhinovirus humain égale ou supérieure à environ 10 fois la quantité exprimée par les cellules HeLa avec un composé à l'essai, et l'étape consistant à déterminer si ledit composé à l'essai bloque la liaison dudit rhinovirus auxdites cellules.

2. Procédé suivant la revendication 1, dans lequel le composé à l'essai est choisi dans le groupe comprenant un peptide, un anticorps ou un de ses fragments.
